Europäisches Patentamt

European Patent Office

Office européen des brevets

(11) Publication number: **0 604 919 A1**

## EUROPEAN PATENT APPLICATION

(21) Application number: **93120827.6**

(22) Date of filing: **23.12.93**

(51) Int. Cl.5: **A61L 9/01**, A61L 15/46, C01G 23/00, C01F 17/00

(30) Priority: **28.12.92 JP 359904/92**

(43) Date of publication of application:
**06.07.94 Bulletin 94/27**

(84) Designated Contracting States:
**AT BE CH DE DK ES FR GB GR IE IT LI LU MC NL PT SE**

(71) Applicant: **ISHIHARA SANGYO KAISHA, LTD.**
**3-15, Edobori 1-chome**
**Nishi-ku, Osaka(JP)**

(72) Inventor: **Maruo, Masatsuyo, c/o Yokkaichi Jigyosho**
**Ishihara Sangyo K.K.,**
**1, Ishiharacho**
**Yokkaichi-shi(JP)**
Inventor: **Watanabe, Mitsuru, c/o Chuo Kenkyusho**
**Ishihara Sangyo K.K.,**
**3-1, Nishishibukawa-2-chome**
**Kusatsu-shi(JP)**

(74) Representative: **Henkel, Feiler, Hänzel & Partner**
**Möhlstrasse 37**
**D-81675 München (DE)**

(54) **Deodorant.**

(57) A deodorant comprises composite particles containing a cerium oxycompound and a titanium oxide. The deodorant is excellent in deodorizing performance for malodorous gases such as ammonia, methyl mercaptan, hydrogen sulfide, trimethylamine, methyl sulfide, acetaldehyde and the like and useful as white deodorants for use in sanitary objects such as paper diaper and sanitary napkins which come into direct contact with skins of human beings.

EP 0 604 919 A1

BACKGROUND OF THE INVENTION

Field of the Invention

The present invention relates to a deodorant capable of adsorbing obnoxious malodorous gases.

Description of Related Art

Obnoxious malodorous gases readily produced in inhabitant environments include ammonia, methyl mercaptan, hydrogen sulfide, trimethylamine, methyl sulfide, and acetaldehyde. An attempt has been made to remove these obnoxious gases maintaining pleasant inhabitant environments, for example, techniques of removing odor by adsorbing malodorous gases on activated carbon or impregnated carbons comprising activated carbon having an acid or alkali deposited have been employed.

These activated carbons are black and so limited in their application. For example, when the activated carbons are incorporated in sanitary products such as paper diapers and sanitary napkins which come into direct contact with the skins of human beings, such treatments as not rendering the products black in color are required so that the products may keep a clean feel, or when the activated carbons are to be incorporated in interior wall sheets, decorations and cosmetics, the products are difficult to impart a desired color so that they are applied to only black commercial items. Moreover, there are problems that the activated carbons has specifically a poor ability in deodorization for ammonia.

On the other hand, there have been marketed a variety of deodorants, for example, white ones capable of imparting a clean feel to the products as well as coloring them in desired tinge such as titanium oxides, silica gel, zeolites, activated alumina and activated clay are commercially available. However, these white deodorants have a low deodorizability to the aforementioned malodorous gases so that they can not be substituted for activated carbons in use. Moreover, such white deodorants can not adsorb malodorous gases at all in the presence of water, because the water is adsorbed in advance. On the other hand, an attempt has been proposed to decompose malodorous gases by irradiating semiconductors such as titanium oxides, cerium oxides and the like with ultraviolet radiation (Japanese Patent KOKAI (Laid-open) No. 2-273513). However, this technique can not be applied to objects which are difficult to expose to ultraviolet radiation, such as paper diaper, sanitary napkins, insoles, undershirts, socks, dishcloths, towels, foot mats, and the like.

SUMMARY OF THE INVENTION

The object of the present invention is to provide a whitish deodorant having an excellent deodorizing performance for malodorous gases.

DETAILED DESCRIPTION OF PREFERRED EMBODIMENTS

The present inventors have made an intensive research about white deodorants, in the course of which a discovery has been made that cerium oxycompounds have an excellent deodorizing ability for malodorous gases, and composite particles comprising the cerium oxycompounds composited with titanium oxides have a more excellent deodorizing ability for malodorous gases, and a deodorant containing at least one of these cerium oxycompounds can retain its deodorizing ability even when it is used in the presence of water, and deodorants containing the cerium oxycompounds are useful for sanitary products such as paper diaper, sanitary napkins, insoles, undershirts, socks, dishcloths, towels, foot mats and the like. The present invention is completed based on the discovery.

The deodorants of the present invention are those containing at least one of cerium oxycompounds. In the present invention, the term "cerium oxycompounds" refers to cerium oxides, hydroxides, hydrated oxides, oxysalts and the like. The cerium hydroxides include cerium compounds having a hydroxyl group such as cerium hydroxide. The cerium oxy salts include cerium compounds having a cerium-oxygen linkage such as cerium oxychloride. These cerium oxycompounds can be produced by any one of known techniques including heating and/or neutralizing cerium compounds such as cerium chloride, nitrate, carbonate, sulfate, oxalate and the like. For example, cerium hydroxides may be produced by adding dropwise an aqueous alkaline solution such as an aqueous sodium hydroxide solution and ammonia water to an aqueous solution of a cerium compound such as cerium chloride with stirring, if necessary, in the presence of hydrogen peroxide, to maintain the resultant mixture basic in nature, or by boiling a solution of cerium in formic acid or in acetic acid with hydrogen peroxide, or by washing cerium ammonium nitrate with cold water. Cerium oxides may be produced, for example, by heating the aforementioned cerium hydroxides, or by heating a cerium carbonate, oxalate, nitrate, or cerium ammonium nitrate at high temperatures. Cerium oxy chloride may be produced by adding water to a product which has been obtained by reducing cerium chloride with sodium metal. The products produced in these ways may be separated, washed, and dried, if necessary. The separation may be accomplished by a conventional technique such as filtration or decantation. The drying may be carried out at an appropriate temperature, suitably at a temperature of 100 to 200 °C.

Although the cerium oxycompounds may be used as such as deodorants in the present invention, they may be used in combination with any one of the materials ordinarily used in the art such as titanium oxides, silicon oxides, aluminum oxide, zeolites and the like. In this case, the content of the cerium oxycompounds can be appropriately selected depending upon the components of the malodorous gases and the concentration thereof, but it should generally be in the range of not less than 0.1 % by weight, preferably 5 to 100 % by weight, most preferably 10 to 100 % by weight expressed as $CeO_2$ by weight. The deodorants of the present invention may be used in the form of particles produced by crashing, pulverizing, or granulating, or of pellets by molding depending upon the circumstances of the end use.

The deodorants comprising composite particles containing the cerium oxycompounds and titanium oxides according to the present invention are described in detail below. The composite particles are not a mere particulate mixture of the cerium oxycompounds and titanium oxides, but such that the particles of the cerium oxycompounds and those of titanium oxides are bonded to each other. The bonded state can be realized so long as the cerium oxycompounds and titanium oxides are physically or chemically in contact with each other. For example, on the surfaces of a single particle or a mass of particles of one of both the cerium oxycompounds and the titanium oxides there may be present locally or uniformly the particles of the other. Alternatively, the surfaces of a single particle or a mass of particles of one of both the cerium oxycompounds and the titanium oxides may be covered in a continuous coating with the particles of the other. Alternatively, the particles of the one may be taken into the interstices in the particles of the other. The composite particles are those being at least partially under one of the aforementioned conditions. The titanium oxides to be used in the present invention include hydrated titanium oxides other than so called titanium dioxide and suboxides.

Though the composition of the composite particles may be varied depending upon target malodorous gases when they are used as deodorants, it should be in the range of a molar ratio of titanium to cerium of Ti : Ce = 99.5 : 0.5 to 10 : 90, preferably 99 : 1 to 30 : 70, most preferably 95 : 5 to 50 : 50. If the amount of the cerium compound is lower than the defined range, the end product will have undesirably a reduced deodorizing performance for malodorous gases when it is used in the presence of water.

The composite particles of the present invention should have a specific surface area of 100 to 400 m$^2$/g, specifically 150 to 400 m$^2$/g to have an excellent deodorizing ability as well as to achieve a good deodorizing speed.

The composite particles can be produced by various techniques, for example, (1) by mixing a cerium compound and a titanium compound with an alkali to neutralize the cerium compound and titanium compound, and then separating and drying the resultant product, (2) by adding a cerium compound and an alkali to a dispersion of titanium oxide to neutralize the cerium compound in the dispersion so that the cerium compound is deposited on the particles of the titanium oxide, and then separating and drying the resultant product. These processes are preferred because the composite particles having excellent characteristics can be easily produced. Particularly, the process (1) is preferred because the deodorants of the present invention can be conveniently and readily produced.

In the aforementioned processes the titanium compounds to be used include, for example, water soluble titanium compounds such as titanium chloride, nitrate, sulfate, and organic acid salts. The cerium compounds to be used include, for example, water soluble cerium compounds such as cerium chloride, nitrate, and sulfate. The alkalis to be used include various ones, for example, sodium hydroxide, potassium hydroxide, and ammonia.

In the process (1), the cerium compound, the titanium compound and the alkali are mixed achieving the resulting mixture at a pH of 8 or more to neutralize the cerium compound and the titanium compound. The neutralization can be achieved by any one of various procedures, for example, by adding an alkali to an aqueous solution of a mixture of the cerium compound and the titanium compound; by adding an alkali to an aqueous solution of the cerium compound to effect a neutralization, followed by adding the titanium compound and an alkali; by adding the cerium compound and the titanium compound to an aqueous alkali solution; or by adding simultaneously the cerium compound, titanium compound and alkali to water, which one can make mention of. The thus produced products may be separated, washed if necessary, and then dried. The separation may be performed by ordinary filtration or decantation or the like. Though the drying may be carried out at an optional temperature, it should suitably be effected at 100 to 200 °C.

The titanium oxide particles to be used in the aforementioned process (2) can be obtained by various known processes. For example, there are i ) a process of hydrolyzing under heat a titanium compound such as titanyl sulfate, titanium chloride and an organic titanium compound, if necessary, in the presence of seed for nucleation, ii) a process of neutralizing a titanium compound such as titanyl sulfate, titanium chloride and an organic titanium compound by adding an alkali thereto, iii) a process of oxidizing titanium chloride, an organic titanium compound and the like in a vapor phase, and iv) a process of firing the titanium oxide obtained in the aforementioned process i), or ii).

In the process (2), first the titanium oxides are dispersed into a medium such as water, classified if necessary, to produce a dispersion. To this dispersion there are added the cerium compound and an alkali achieving the resulting mixture at a pH of 8 or more to neutralize the cerium compound in the titanium oxide dispersion. The neutralization can be achieved by any one of various procedures, for example, by adding simultaneously the cerium compound and the alkali to the titanium oxide dispersion; by adding the cerium compound to the titanium oxide dispersion, followed by adding the alkali; or by adding the alkali to the titanium oxide dispersion, followed by adding the cerium compound, which one can make mention of. The thus produced products may be separated, washed if necessary, and then dried. The separation may be performed by ordinary filtration or decantation or the like. Though the drying may be carried out at an optional temperature, it should suitably be effected at 100 to 200 °C.

In the processes (1) and (2), the conditions such as the concentrations of the cerium compound, titanium compound, and alkali, the addition speed, the temperature of the neutralization reaction, and the concentration of the titanium oxides in the dispersion are not critical, but can be appropriately selected. Ammonium salts such as urea and ammonium chloride may be used in order to control the rate of neutralization. Moreover, dispersants such as orthophosphoric acid, pyrophosphoric acid, hexametaphosphoric acid, alkali salts thereof, sodium orthosilicate, and sodium metasilicate may be added to the titanium oxide dispersion so long as the deodorizing performance of the resulting composite particles is not adversely affected.

Though the thus produced composite particles are ready to use as deodorants, they may be used in combination with titanium oxides, silicon oxides, aluminum oxide and/or zeolites similarly to the case of the cerium compounds as described above. In this case the content of the composite particles can be appropriately selected depending upon the components of the malodorous gases and the concentration thereof, and generally it is in the range of not less than 0.1 % by weight, preferably 5 to 100 % by weight, more preferably 10 to 100 % by weight expressed as the total weight of $TiO_2$ and $CeO_2$. The composite particles may be used in the form of powder, granule, or pellet after deagglomeration, pulverization, or molding depending upon the circumstances of the end use.

Moreover, other additives may be incorporated in the cerium oxycompound particles and the composite particles, comprising the cerium oxycompound and the titanium oxide, if necessary, in order to enhance the deodorant performance and/or control the hydrophilic or oleophilic property of the particles. The additives include, for example, inorganic materials such as titanium oxides, silicon oxides, aluminum oxide, zinc oxide, inorganic acids, inorganic alkalis; organic materials such as ascorbic acid, vegetable oils, water absorptive polymers, oil absorptive polymers, organic acids, organic alkalis which can be selected for use depending upon the circumstances of the end use.

When the deodorants of the present invention are used for paper diaper and sanitary napkins, they should preferably be mixed in an amount of 0.5 to 80 % by weight based on the weight of water absorptive polymer. When they are used for insoles, they should preferably be dispersed in pores in foamed polymers. When they are used for undershirts and the like, they should preferably be deposited on the surfaces of fibers, or kneaded into the fibers, followed by subjecting to etching treatment.

Example 1

To 260 milliliters of an aqueous 0.1 mol/liter cerium trichloride solution, an aqueous 1 N sodium hydroxide solution was added dropwise with stirring at room temperature to a pH of 9 to produce a product. Then the product was filtered, washed, dried at 105 °C, and then ground with an agate mortar and pestle to yield a deodorant of the present invention (Sample A).

Example 2

600 milliliters of water were charged in a reactor, and a mixture of 200 milliliters of an aqueous 0.1 mol/liter titanium tetrachloride solution and 200 milliliters of an aqueous 0.1 mol/liter cerium trichloride

4

solution were added to the reactor together with an aqueous 2 N sodium hydroxide solution at room temperature while maintaining a pH of the dispersion at 9 for 50 minutes to produce a product. Subsequently, the product was filtered, washed, dried at 105 °C, and then ground with an agate mortar and pestle to yield a deodorant of the present invention (Sample B).

Example 3

One liter of an aqueous 2 mol/liter titanyl sulfate solution was hydrolyzed under heat in the presence of seed for nucleation to produce a precipitate. Subsequently the precipitate was filtered, washed, and dried at 120 °C to produce a titanium oxide.

After 80 grams of the titanium oxide were dispersed in one liter of pure water, 110 milliliters of an aqueous 0.1 mol/liter cerium trichloride solution were added dropwise to the dispersion together with an aqueous 6 N sodium hydroxide solution with stirring at room temperature while maintaining a pH of the dispersion at 9 for 10 minutes to produce a product. Subsequently, the product was filtered, washed, dried at 105 °C, and then ground with an agate mortar and pestle to yield a deodorant of the present invention (Sample C).

Example 4

The procedure identical to that in Example 3 was conducted, except that the aqueous cerium trichloride solution was used in an amount of 53 milliliters, to yield a deodorant of the present invention (Sample D).

Example 5

The procedure identical to that in Example 3 was conducted, except that the aqueous cerium trichloride solution was used in an amount of 53 milliliters and 6 mols of ammonium chloride were added to the aqueous cerium trichloride solution, to yield a deodorant of the present invention (Sample E).

Comparative Example 1

The titanium oxide produced in Example 3 without effecting the treatment with the cerium oxide compound was used as Sample F.

The specific surface areas and the adsorption rates of the Samples A to F obtained in Examples and Comparative Example are set forth in Table 1.

The Samples were evaluated for the adsorption of malodorous gases according to the following procedure. First each of methyl mercaptan, hydrogen sulfide and ammonia was diluted with nitrogen gas to about 1000 ppm. Then one liter of the diluted gas was introduced into a polyester bag containing 0.1 gram of the Sample and after sealed, left to stand for 5 hours. Thereafter, the concentration of the malodorous gases remaining in the bag was measured by a gas chromatography or a gas sensor and the adsorption rates were calculated from the concentration values of the diluted gases introduced.

Table 1

| Example | Sample | Molar ratio Ti : Ce | Particle color | Specific surface area (m²/g) | Gas adsorption (%) | | |
|---|---|---|---|---|---|---|---|
| | | | | | Methyl mercaptan | Hydrogen sulfide | Ammonia |
| 1 | A | 0 : 100 | Yellow | 79 | 73.6 | 100 | 65.0 |
| 2 | B | 50 : 50 | Pale Yellow | 290 | 99.8 | 100 | 92.5 |
| 3 | C | 90 : 10 | White | 176 | 79.0 | 100 | 95.0 |
| 4 | D | 95 : 5 | White | 224 | 97.4 | 100 | 90.0 |
| 5 | E | 95 : 5 | White | 224 | 85.0 | 100 | 90.0 |
| Comp. Ex. | F | 100 : 0 | White | 290 | 10.7 | 10 | 100 |

Next, one gram of each of the Samples (B, D, F) obtained in Examples and Comparative Example was added to 100 grams of a high molecular weight water absorptive polymer (AQUALIC CAK-4, available from NIPPON SHOKUBAI Co.), and thereafter, an aliquot of one gram from each sample mixture was placed in a polyester bag. Then, 30 milliliters of water and 200 milliliters of a gas mixture containing methyl mercaptan in a concentration of 1000 ppm in 800 milliliters of air and nitrogen gas were added to the polyester bag which, after sealed, was left to stand for 5 hours. Thereafter, the concentration of the methyl mercaptan remaining in the bag was measured by a gas chromatography and the adsorption rates were calculated from the concentration values of the diluted gases introduced. The results are shown in Table 2.

Table 2

|  | Sample | Gas adsorption (%) |
|---|---|---|
|  |  | Methyl mercaptan |
| Example 2 | B | 36.3 |
| Example 4 | D | 39.2 |
| Comp. Example | F | 0 |

The deodorants of the present invention comprising the cerium oxycompounds are excellent in deodorizing performance for malodorous gases such as ammonia, methyl mercaptan, hydrogen sulfide, trimethylamine, methyl sulfide, acetaldehyde and the like and capable of retaining their deodorizing performance even in the presence of water in use and useful as whitish deodorants for use in sanitary objects such as paper diaper and sanitary napkins which come into direct contact with skins of human beings.

The deodorants comprising the composite particles containing the cerium oxycompounds composited with the titanium oxides according to the present invention retains the deodorizing ability of the cerium oxycompounds and in addition can enhance the deodorizing performances for other malodorous gases. They are superior in deodorant performance for malodorous gases to a mere mixture of the cerium oxycompounds and the titanium oxides and very suitable as white deodorants because the color of the particles is white. Moreover, the deodorants of the present invention are excellent in durability to acid and base and capable of retaining their deodorant performance even in the presence of water in use so that they are suitable as white deodorants in various applications.

Moreover, the composite particles comprising the cerium oxycompound and titanium oxides produced by mixing the cerium compound, titanium compound, and alkali to neutralize the cerium compound and the titanium compound, separating and drying the resultant product. They can be used for pigments, catalysts, photocatalysts, catalyst carriers, fillers,ultraviolet radiation absorbents, materials for ceramics and the like other than deodorants so that they are extremely useful in industry.

## Claims

1. A deodorant capable of adsorbing malodorous gases comprising a cerium oxycompound as effective component.

2. The deodorant according to Claim 1, wherein said cerium oxycompound is included in an amount of not less than 0.1 % by weight expressed as $CeO_2$.

3. The deodorant according to Claim 1, wherein said cerium oxycompound is included in an amount of 5 to 100 % by weight expressed as $CeO_2$.

4. The deodorant according to Claim 1, wherein other component than said cerium oxycompound is titanium oxide.

5. A deodorant capable of adsorbing malodorous gases comprising composite particles containing a cerium oxycompound and a titanium oxide as effective component.

6. The deodorant according to Claim 5, wherein said composite particles have a molar ratio of titanium to cerium, Ti : Ce of 99.5 : 0.5 to 10 : 90.

7. The deodorant according to Claim 5, wherein said composite particles have a molar ratio of titanium to cerium, Ti : Ce of 99 : 1 to 30 : 70.

8. The deodorant according to Claim 5, wherein said composite particles have a specific surface area of 100 to 400 $m^2$/g.

9. A sanitary product comprising the deodorant according to any one of Claims 1 through 8 as effective component.

10. A process for producing composite particles comprising the steps of mixing a cerium compound, a titanium compound and an alkali to neutralizing said cerium compound and said titanium compound, then separating and drying the resultant product.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | DATABASE WPI<br>Week 8845,<br>Derwent Publications Ltd., London, GB;<br>AN 88-319394<br>& JP-A-63 236 541 (NISSAN MOTOR KK) 3 October 1988<br>* abstract * | 1-7 | A61L9/01<br>A61L15/46<br>C01G23/00<br>C01F17/00 |
| X | US-A-4 537 865 (S. OKABE)<br>* claims; example 6 * | 10 | |
| A | EP-A-0 239 479 (RHONE-POULENC) | 10 | |
| A | US-A-5 108 739 (T. KURIHARA) | 9 | |

TECHNICAL FIELDS
SEARCHED      (Int.Cl.5)

A61L
C01G
C01F

The present search report has been drawn up for all claims

| Place of search | Date of completion of the search | Examiner |
|---|---|---|
| THE HAGUE | 29 March 1994 | Cousins-Van Steen, G |

EPO FORM 1503 03.82 (P04C01)